# EUROPÄISCHE PATENTSCHRIFT

(11) **EP 2 953 654 B1**
(45) Veröffentlichungstag und Bekanntmachung des Hinweises auf die Patenterteilung: **26.10.2016**
(21) Anmeldenummer: 14700813.0
(22) Anmeldetag: 03.01.2014
(51) Int. Cl.: A61L 2/07, A61L 2/04, B01J 3/04, F28D 20/00

(54) **STERILISATIONSVORRICHTUNG UND STERILISATIONSVERFAHREN MIT ENERGIERÜCKGEWINNUNG**
STERILISING DEVICE AND STERILISATION METHOD HAVING ENERGY RECOVERY
DISPOSITIF DE STÉRILISATION ET PROCÉDÉ DE STÉRILISATION AVEC RÉCUPÉRATION D'ÉNERGIE

(30) Priorität: 11.02.2013 DE 102013202188
(43) Veröffentlichungstag der Anmeldung: 16.12.2015
(73) Patentinhaber: Robert Bosch GmbH, 70442 Stuttgart (DE)
(72) Erfinder: GRABNER, Wilfried, A-2630 Ternitz (AT); GYOEROEG, Erich, A-7431 Bad Tatzmannsdorf (AT); HASSAN, Mahmoud, A-1120 Wien (AT)
(86) Internationale Anmeldenummer: PCT/EP2014/050033
(87) Internationale Veröffentlichungsnummer: WO 2014/121953

(56) Entgegenhaltungen:
- EP-A1- 2 289 615
- DE-A1- 4 442 709
- DE-C1- 4 312 474
- US-A- 4 708 849

## Beschreibung

### Stand der Technik

Die vorliegende Erfindung betrifft eine Vorrichtung und ein Verfahren zum Sterilisieren von Produkten mit Heißwasser und/oder Heißdampf.

DE 44 42 709 A1 zeigt eine Sterilisationsvorrichtung gemäß dem Oberbegriff des Anspruchs 1. Weiterer relevanter Stand der Technik ist den Druckschriften US 4 708 849 A und EP 2 289 615 A1 zu entnehmen.

Die terminale Sterilisation, insbesondere die Heißwasserberieselung, wird in der Regel recht groß, beispielsweise mit Volumen von über 5m³, ausgeführt, um eine möglichst große Menge an Produkten pro Lauf sterilisieren zu können. Die Laufzeit kann über die Menge an verfügbaren Medien, die Größe der Wärmetauscher und die verfügbare Temperatur der Heiz- bzw. Kühlmedien beeinflusst werden. Die Auslegung dieser Komponenten wiederum ist von der Beladungsdichte, vom Produkt und von der erforderlichen Prozessdauer, aber auch von der Größe und Masse des Sterilisators, die mitgeheizt bzw. mitgekühlt werden muss, abhängig. Mit der andauernden Kühlung und Heizung der Sterilisationsvorrichtung ist ein sehr hoher Energieverbrauch verbunden.

### Offenbarung der Erfindung

Mit der Sterilisationsvorrichtung gemäß Anspruch 1 und dem Sterilisationsverfahren gemäß Anspruch 8 ist es möglich, den Energieverbrauch für das Sterilisieren deutlich zu verringern. Beim Heizen des Fluides für die Kammer zum Sterilisieren sind Energieeinsparungen bis zu 40% und beim Kühlen bis zu 60% möglich. Dies wird erreicht durch eine Sterilisationsvorrichtung umfassend zumindest eine Kammer zum Sterilisieren von Produkten und einen primären, an die Kammer angeschlossenen Fluidkreislauf. Der primäre Fluidkreislauf dient zum Beaufschlagen der Kammer bzw. der Produkte mit Heißwasser und/oder Heißdampf. Des Weiteren umfasst die Sterilisationsvorrichtung einen sekundären Fluidkreislauf und einen zweiten Wärmetauscher. Der sekundäre Fluidkreislauf ist über den zweiten Wärmetauscher mit dem primären Fluidkreislauf verbunden, so dass mittels des sekundären Fluidkreislaufes der primäre Fluidkreislauf erwärmt und/oder gekühlt werden kann. Des Weiteren umfasst die Sterilisationsvorrichtung einen Schichtspeichertank im sekundären Fluidkreislauf. Der Schichtspeichertank umfasst mehrere Temperaturzonen, wobei die einzelnen Temperaturzonen mit dem Fluid des sekundären Fluidkreislaufes separat be- und entladbar sind. Zum Kühlen des primären Fluidkreislaufes wird also mittels des zweiten Wärmetauschers dem primären Fluidkreislauf Wärme entzogen. Dabei erfolgt eine Erwärmung des Fluides im sekundären Fluidkreislauf. Diese Wärme kann in den Temperaturzonen des Schichtspeichertanks gespeichert werden. Beim anschließenden Erhitzen des Fluides im primären Fluidkreislauf wird die Wärme aus dem Schichtspeichertank wieder entnommen und kann über den zweiten Wärmetauscher an den primären Fluidkreislauf übertragen werden. Die einzelnen Temperaturzonen des Schichtspeichertanks ermöglichen ein Vorhalten von Fluid mit verschiedensten Temperaturen. Durch ein Zusammenmischen oder eine schichtweise Entnahme des Fluides aus den einzelnen Temperaturzonen wird der primäre Fluidkreislauf unterstützt um auf die gewünschte Temperatur erhitzt oder abgekühlt zu werden. Der primäre Fluidkreislauf umfasst vorzugsweise einen ersten Wärmetauscher. Der erste Wärmetauscher ist an eine unabhängige Heiz- und Kühlleitung angeschlossen, sodass der primäre Fluidkreislauf auch unabhängig vom sekundären Fluidkreislauf betrieben werden kann. Durch die Verwendung des erfindungsgemäßen Schichtspeichertanks kann der Energieverbrauch der Sterilisationsvorrichtung signifikant gesenkt werden.

Die Unteransprüche zeigen bevorzugte Weiterbildungen der Erfindung.

Vorzugsweise ist ein dritter Wärmetauscher vorgesehen. Dieser dritte Wärmetauscher dient zum zusätzlichen Kühlen des Fluides im sekundären Fluidkreislauf, wobei der dritte Wärmetauscher an einen externen Kühlkreislauf angeschlossen ist.

Insbesondere ist vorgesehen, dass der dritte Wärmetauscher in den Schichtspeichertank integriert ist. Insbesondere ist der dritte Wärmetauscher dabei in die Temperaturzone mit der niedrigsten Temperatur, also insbesondere die unterste Temperaturzone, eingebaut. Mittels des integrierten dritten Wärmetauschers ist es möglich, das Fluid direkt in dem Schichtspeichertank und/oder beim Herausfließen aus dem Schichtspeichertank zu kühlen um damit den Wirkungsgrad der Energieeinsparrung beim Kühlen zu erhöhen.

Der Schichtspeichertank umfasst zu den Temperaturzonen jeweils einen eigenen, steuerbaren Anschluss. Über diesen steuerbaren Anschluss kann Fluid direkt in die einzelnen Temperaturzonen be- und entladen werden. Die steuerbaren Anschlüsse dienen somit bei der Entnahme von Fluid aus dem Schichtspeichertank zum Zusammenmischen der gewünschten Temperatur oder zur schichtweisen Entnahme. Beim Beladen des Schichtspeichertanks wird das Fluid über die steuerbaren Anschlüsse in die richtige Temperaturzone geleitet.

Der Schichtspeichertank ist insbesondere zur Aufnahme einer Fluidsäule ausgebildet. Die unterschiedlichen Temperaturzonen sind dabei in der Fluidsäule übereinander oder nebeneinander angeordnet.

Um ein Vermischen des Fluides unterschiedlicher Temperaturzonen weitestgehend zu vermeiden, sind in dem Schichtspeichertank Trennvorrichtungen vorgesehen. Diese Trennvorrichtungen müssen nicht zwangsläufig die einzelnen Temperaturzonen vollständig voneinander abtrennen. In erster Linie dienen die Trennvorrichtungen zur Vermeidung einer turbulenten Durchmischung des Fluides verschiedener Temperaturzonen.

Der Schichtspeichertank umfasst zumindest drei, vorzugsweise zumindest vier Temperaturzonen, die separat be- und entladbar sind.

Über den sekundären Fluidkreislauf mit einem Schichtspeichertank können bevorzugt auch mehrere Kammern mit jeweils einem primären Fluidkreislauf versorgt werden. Dabei ist pro primärem Fluidkreislauf ein zweiter Wärmetauscher vorgesehen, der an den sekundären Fluidkreislauf angeschlossen ist.

Des Weiteren ist bevorzugt eine Steuervorrichtung vorgesehen, die den Volumenstrom in den einzelnen Fluidleitungen, insbesondere in den Anschlüssen des Schichtspeichertanks steuert und/oder regelt.

Die Erfindung umfasst des Weiteren ein Sterilisationsverfahren mit den folgenden Schritten: (i) Sterilisieren von Produkten in einer Kammer mit Heißwasser und/oder Heißdampf aus einem primären Fluidkreislauf, (ii) Wahlweises Erwärmen oder Kühlen des primären Fluidkreislaufes mit einem sekundären Fluidkreislauf, und (iii) Speichern des Fluides des zweiten Fluidkreislaufes in unterschiedlichen Temperaturzonen.

Die Unteransprüche und die vorteilhaften Ausgestaltungen der erfindungsgemäßen Sterilisationsvorrichtung finden entsprechend vorteilhafte Anwendung auf das erfindungsgemäße Sterilisationsverfahren.

Insbesondere ist vorgesehen, dass zum Erwärmen und/oder Kühlen des primären Fluidkreislaufes das Fluid des sekundären Fluidkreislaufes aus den unterschiedlichen Temperaturzonen zusammengemischt oder schichtweise entnommen wird. Dementsprechend ist auch bevorzugt vorgesehen, dass ein vom zweiten Wärmetauscher kommender Rücklauf entsprechend auf die unterschiedlichen Temperaturzonen aufgeteilt wird.

Des Weiteren ist bevorzugt vorgesehen, dass das gespeicherte Fluid, vorzugsweise in der Temperaturzone mit niedrigster Temperatur, mittels eines Kühlkreislaufes zusätzlich gekühlt werden kann.

### Kurze Beschreibung der Zeichnung

Nachfolgend wird ein Ausführungsbeispiel der Erfindung unter Bezugnahme auf die begleitende Zeichnung im Detail beschrieben. Dabei zeigt:

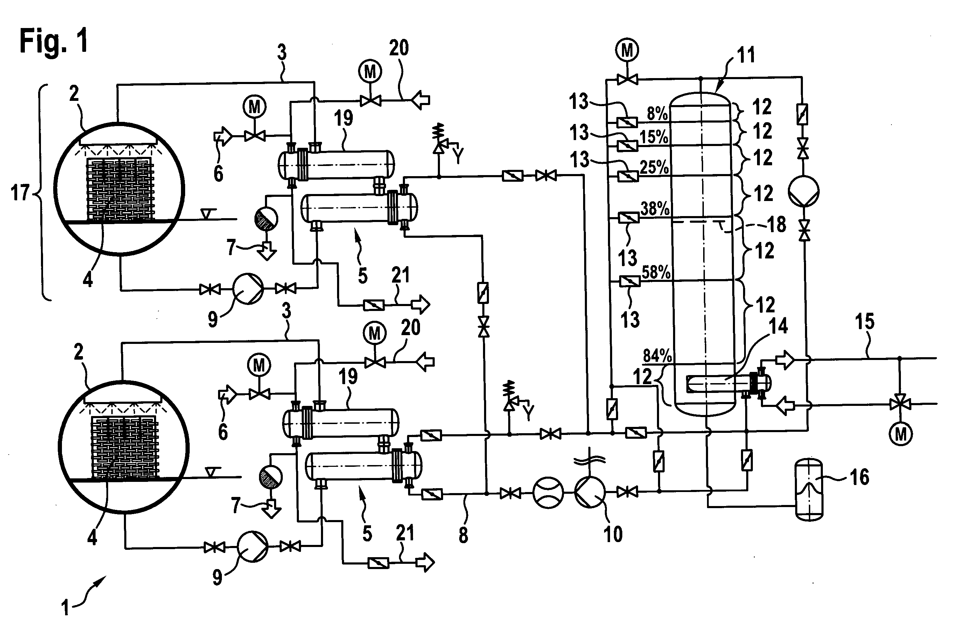

- Figur 1: ein Schaltbild einer erfindungsgemäßen Sterilisationsvorrichtung gemäß einem Ausführungsbeispiel.

### Ausführungsform der Erfindung

Fig. 1 zeigt ein Schaltbild einer Sterilisationsvorrichtung 1 gemäß dem Ausführungsbeispiel. Die Sterilisationsvorrichtung 1 umfasst eine Kammer 2. In dieser Kammer 2 sind zu sterilisierende Produkte 4 angeordnet. Die Kammer 2 ist an einen primären Fluidkreislauf 3 angeschlossen. In diesem primären Fluidkreislauf 3 wird das Fluid mittels einer ersten Pumpe 9 gefördert und mittels eines Wärmetauschers 19 erhitzt oder abgekühlt. Mittels des primären Fluidkreislaufes 3 werden die Produkte 4 in der Kammer mit Heißwasser berieselt. An dem ersten Wärmetauscher 19 sind ein Dampfzulauf 6, eine Kühlmedienzuleitung 20, eine Kühlmedienrückleitung 21 und ein Kondensatablauf 7 ausgebildet.

Des Weiteren ist ein zweiter Wärmetauscher 5 vorgesehen. Der zweite Wärmetauscher 5 verbindet den primären Fluidkreislauf 3 mit einem sekundären Fluidkreislauf 8. Der sekundäre Fluidkreislauf 8 dient zum Erwärmen und Kühlen des primären Fluidkreislaufes 3. Dieser Wärmeaustausch erfolgt über den zweiten Wärmetauscher 5.

Das Fluid, vorzugsweise Wasser, im sekundären Fluidkreislauf 8 wird mittels einer zweiten Pumpe 10 gefördert. Diese Pumpe kann in ihrer Leistung individuell geregelt werden.

Der sekundäre Fluidkreislauf 8 umfasst des Weiteren einen Schichtspeichertank 11. In diesem Schichtspeichertank 11 befindet sich eine Fluidsäule, die in unterschiedliche, übereinander oder nebeneinander angeordnete Temperaturzonen 12 unterteilt ist. Zu den Temperaturzonen 12 führen einzelne Anschlüsse 13. Über diese Anschlüsse 13 können die einzelnen Temperaturzonen 12 des Schichtspeichertanks 11 be- und entladen werden. Die Anschlüsse 13 umfassen hierzu Absperrventile oder Mischventile, die über eine Steuerungseinrichtung gesteuert oder geregelt werden.

Des Weiteren sind zwischen den Temperaturzonen 12 Trennvorrichtungen 18 vorgesehen, die ein turbulentes Durchmischen des Fluides in dem Schichtspeichertank 11 weitestgehend vermeiden.

Mittels der einzelnen Anschlüsse 13 kann das Fluid für den sekundären Fluidkreislauf 8 auf die entsprechende Temperatur zusammengemischt oder schichtweise entnommen werden. Dadurch ist ein gezieltes Erwärmen und Abkühlen des primären Fluidkreislaufes 3 über den zweiten Wärmetauscher 5 möglich.

Für eine zusätzliche Kühlung des sekundären Fluidkreislaufes 8 ist ein dritter Wärmetauscher 14 vorgesehen. Der dritte Wärmetauscher 14 ist in den Schichtspeichertank 11, insbesondere in die unterste Temperaturzone 12, integriert. Der dritte Wärmetauscher 14 ist an einen externen Kühlkreislauf 15 angeschlossen.

Des Weiteren ist eine Druckausgleichsvorrichtung 16 im sekundären Fluidkreislauf 8 vorgesehen.

Die Darstellung in Fig. 1 zeigt eine weitere Anordnung 17, die ebenfalls einen primären Fluidkreislauf 3, eine Kammer 2 und einen ersten Wärmetauscher 19 aufweist. An den sekundären Fluidkreislauf 8 können mehrere dieser weiteren Anordnungen 17 angeschlossen werden.

Nicht gezeigt in Fig. 1 ist eine Heizvorrichtung, beispielsweise ein Heizkessel, zum zusätzlichen Erhitzen des sekundären Fluidkreislaufes 8. Im ersten Sterilisationszyklus ist die volle Energie für das Heizen der Kammer 2 nötig. Die Wassertemperatur im primären Fluidkreislauf 8 beträgt in der Sterilisationsphase ca. 121 °C.

Der Schichtspeichertank 11 ist stets mit dem Fluid des zweiten Fluidkreislaufes 8, insbesondere mit Wasser, gefüllt, wobei die Temperatur des Fluides von unten nach oben steigt. Die Trennvorrichtungen 18 im Schichtspeichertank 11 verhindern eine schnelle Vermischung der einzelnen Temperaturzonen.

Beim Start des Kühlvorgangs zum Kühlen des primären Fluidkreislaufes 3 wird das Fluid des sekundären Fluidkreislaufes 8 durch das heiße Prozesswasser im primären Fluidkreislauf 3 aufgeheizt. Die Energie aus dem primären Fluidkreislauf 3 wird somit über den zweiten Wärmetauscher 5 an den sekundären Fluidkreislauf 8 übertragen und kann im Schichtspeichertank 11 gespeichert werden. Das heiße Fluid im sekundären Fluidkreislauf 8 wird in Abhängigkeit von der Temperatur von oben beginnend in den Schichtspeichertank 11 über die verschiedenen Anschlüsse 13 geladen.

Zusätzlich wird die unterste Temperaturzone 12 mit dem dritten Wärmetauscher 14 gekühlt. Dies dient dazu, dass der primäre Fluidkreislauf 3 möglichst schnell abgekühlt werden kann.

Beim nächsten Lauf, also beim nächsten Aufheizen der Kammer 2, wird das heiße Fluid schichtweise aus dem Schichtspeichertank 11 entnommen. Dies passiert schichtweise, bis kein positiver Energieeintrag in den primären Fluidkreislauf 3 mehr geleistet werden kann. Ab diesen Zeitpunkt wird der primäre Fluidkreislauf 3 über die Dampfleitung 6 weiter erwärmt bist die Zieltemperatur erreicht ist.

Beim Kühlen ist der Wirkungsgrad höher, da der dritte Wärmetauscher 14 im Schichtspeichertank 11 zusätzlich unterstützend wirkt. Der Energieaufwand für den dritten Wärmetauscher 14 ist geringer als der Energieaufwand im herkömmlichen Kühlprozess. Es besteht die Möglichkeit nur über den dritten Wärmetauscher 14 und den Schichtspeichertank 11 zu kühlen oder aber zusätzlich über die Kühlmedienzuleitung 20, was den zusätzlichen Vorteil hat, bei Ausfall eines Systems auf das andere wechseln zu können. Im herkömmlichen Prozess wird die gesamte Energie über ein Kühlwassersystem, üblicherweise einem geschlossenen Kühlkreislauf, aus dem primären Fluidkreislauf 3 entfernt. Das Kühlwassersystem selbst wird wiederum über Kühlaggregate oder Kühltürme gekühlt. Durch die Einsparung an Kühlenergie kann einerseits die bauseitige Investition in das Kühlsystem geringer ausfallen und andererseits sind auch die laufenden Kosten entsprechend geringer.

Die aufgezeigte Sterilisationsvorrichtung 1 sowie das zugehörige Sterilisationsverfahren können für unterschiedlichste Kammern 2 verwendet werden. Durch eine intelligente Steuerung wird sichergestellt, dass der Schichtspeichertank 11 stets voll ist. Wenn Fluid aus einer Temperaturzone 12 entnommen wird, wird gleichzeitig Fluid in eine andere Temperaturzone 12 gepumpt, so dass das Gesamtvolumen im Schichtspeichertank 11 gleich bleibt. Hierzu erfolgt vorzugsweise eine intelligente Steuerung der Abläufe mittels steuerbaren Ventilen und Temperatursensoren in der Verrohrung, im Schichtspeichertank 11, in den Wärmetauschern 5, 14, 19 und/oder in der Kammer 2.

## Patentansprüche

1. Sterilisationsvorrichtung (1) umfassend
- zumindest eine Kammer (2) zum Sterilisieren von Produkten (4),
- einen primären, an die Kammer (2) angeschlossenen Fluidkreislauf (3) zum Beaufschlagen der Kammer mit Heißwasser und/oder Heißdampf, und
- einen sekundären Fluidkreislauf (8) zum Erwärmen und/oder Kühlen des primären Fluidkreislaufes (3), wobei der sekundäre Fluidkreislauf (8) über einen zweiten Wärmetauscher (5) mit dem primären Fluidkreislauf (3) verbunden ist,
**gekennzeichnet durch**
- einen Schichtspeichertank (11) im sekundären Fluidkreislauf (8) mit mehreren Temperaturzonen (12), wobei die Temperaturzonen (12) mit dem Fluid des sekundären Fluidkreislaufes (8) separat be- und entladbar sind.

2. Sterilisationsvorrichtung nach Anspruch 1, **gekennzeichnet durch** einen dritten Wärmetauscher (14) zum Kühlen des Fluides im sekundären Fluidkreislauf (8), wobei der dritte Wärmetauscher (14) an einen Kühlkreislauf (15) angeschlossen ist.

3. Sterilisationsvorrichtung nach Anspruch 2, **dadurch gekennzeichnet, dass** der dritte Wärmetauscher (14) in den Schichtspeichertank (11), vorzugsweise in die Temperaturzone (12) mit niedrigster Temperatur, integriert ist.

4. Sterilisationsvorrichtung nach einem der vorhergehenden Ansprüche, **dadurch gekennzeichnet, dass** der Schichtspeichertank (11) zu mehreren Temperaturzonen (12) jeweils einen eigenen, steuerbaren Anschluss (13) zum Be- und Entladen der jeweiligen Temperaturzone (12) umfasst.

5. Sterilisationsvorrichtung nach einem der vorhergehenden Ansprüche, **dadurch gekennzeichnet, dass** der Schichtspeichertank (11) zur Aufnahme einer Fluidsäule ausgebildet ist, wobei die Temperaturzonen (12) in der Fluidsäule übereinander oder nebeneinander angeordnet sind.

6. Sterilisationsvorrichtung nach Anspruch 5, **dadurch gekennzeichnet, dass** in dem Schichtspeichertank (11) zumindest eine Trennvorrichtung (18) angeordnet ist, um eine turbulente Durchmischung des Fluides verschiedener Temperaturzonen (12) zu vermeiden.

7. Sterilisationsvorrichtung nach einem der vorhergehenden Ansprüche, **dadurch gekennzeichnet, dass** der Schichtspeichertank (11) zumindest drei, vorzugsweise zumindest vier, Temperaturzonen (12) umfasst, die mittels einzelnen Anschlüssen (13) separat be- und entladbar sind.

8. Sterilisationsverfahren unter Verwendung einer Sterilisationsvorrichtung nach einem der vorhergehenden Ansprüche, umfassend die folgenden Schritte:
- Sterilisieren von Produkten (4) in einer Kammer (2) mit Heißwasser und/oder Heißdampf aus einem primären Fluidkreislauf (3),
- wahlweises Erwärmen oder Kühlen des primären Fluidkreislaufes (3) mit einem sekundären Fluidkreislauf (8), und
- Speichern des Fluides des zweiten Fluidkreislaufes (8) in unterschiedlichen Temperaturzonen (12).

9. Sterilisationsverfahren nach Anspruch 8, **dadurch gekennzeichnet, dass** zum Erwärmen und/oder Kühlen des primären Fluidkreislaufes (3) das Fluid des sekundären Fluidkreislaufes (8) aus den unterschiedlichen Temperaturzonen (12) zusammengemischt wird und/oder schichtweise entnommen wird.

10. Sterilisationsverfahren nach einem der Ansprüche 8 oder 9, **dadurch gekennzeichnet, dass** das gespeicherte Fluid, vorzugsweise in der Temperaturzone (12) mit niedrigster Temperatur, mittels eines Kühlkreislaufes (15) gekühlt werden kann.

## Claims

1. Sterilization device (1), comprising
- at least one chamber (2) for the sterilization of products (4),
- a primary fluid circuit (3) which is connected to the chamber (2) and which serves for charging the chamber with hot water and/or hot vapour, and
- a secondary fluid circuit (8) for the heating and/or cooling of the primary fluid circuit (3), wherein the secondary fluid circuit (8) is connected to the primary fluid circuit (3) by way of a second heat exchanger (5),
**characterized by**
- a stratified store tank (11) in the secondary fluid circuit (8) with multiple temperature zones (12), wherein the temperature zones (12) can be separately charged with the fluid of the secondary fluid circuit (8) and discharged.

2. Sterilization device according to Claim 1, **characterized by** a third heat exchanger (14) for the cooling of the fluid in the secondary fluid circuit (8), wherein the third heat exchanger (14) is connected to a cooling circuit (15).

3. Sterilization device according to Claim 2, **characterized in that** the third heat exchanger (14) is integrated into the stratified store tank (11), preferably into the temperature zone (12) with the lowest temperature.

4. Sterilization device according to one of the preceding claims, **characterized in that** the stratified store tank (11) comprises, for multiple temperature zones (12), in each case one dedicated, controllable port (13) for the charging and discharging of the respective temperature zone (12).

5. Sterilization device according to one of the preceding claims, **characterized in that** the stratified store tank (11) is designed for accommodating a fluid column, wherein the temperature zones (12) are arranged one above the other or adjacent to one another in the fluid column.

6. Sterilization device according to Claim 5, **characterized in that**, in the stratified store tank (11), there is arranged at least one separating device (18) for preventing turbulent mixing of the fluid of different temperature zones (12).

7. Sterilization device according to one of the preceding claims, **characterized in that** the stratified store tank (11) comprises at least three, preferably at least four, temperature zones (12) which can be charged and discharged separately by way of individual ports (13).

8. Sterilization method using a sterilization device according to one of the preceding claims, comprising the following steps:
- sterilizing products (4) in a chamber (2) with hot water and/or hot steam from a primary fluid circuit (3),
- selectively heating or cooling the primary fluid circuit (3) by way of a secondary fluid circuit (8), and
- storing the fluid of the second fluid circuit (8) in different temperature zones (12).

9. Sterilization method according to Claim 8, **characterized in that**, for the heating and/or cooling of the primary fluid circuit (3), the fluid of the secondary fluid circuit (8) is mixed together, and/or extracted in stratified fashion, from the different temperature zones (12).

10. Sterilization method according to either of Claims 8 and 9, **characterized in that** the stored fluid can, preferably in the temperature zone (12) with the lowest temperature, be cooled by way of a cooling circuit (15).

## Revendications

1. Ensemble de stérilisation (1) comportant :
- au moins une chambre (2) permettant de stériliser des produits (4),
- un circuit primaire (3) de fluide raccordé à la chambre (2) et qui applique sur la chambre de l'eau chaude et/ou de la vapeur chaude,
- un circuit secondaire (8) de fluide qui réchauffe et/ou refroidit le circuit primaire (3) de fluide, le circuit secondaire (8) de fluide étant raccordé au circuit primaire (3) de fluide par un deuxième échangeur de chaleur (5),
**caractérisé par**
- une cuve (11) d'accumulation stratifiée prévue dans le circuit secondaire (8) de fluide et présentant plusieurs zones de température (12), les zones de température (12) pouvant être chargées et déchargées séparément par le fluide du circuit secondaire (8).

2. Ensemble de stérilisation selon la revendication 1, **caractérisé par** un troisième échangeur de chaleur (14) qui refroidit le fluide du circuit secondaire (8), le troisième échangeur de chaleur (14) étant raccordé à un circuit de refroidissement (15).

3. Ensemble de stérilisation selon la revendication 2, **caractérisé en ce que** le troisième échangeur de chaleur (14) est intégré dans la cuve (11) à accumulation stratifiée, de préférence dans la zone de température (12) qui présente la température la plus basse.

4. Ensemble de stérilisation selon l'une des revendications précédentes, **caractérisé en ce que** la cuve (11) d'accumulation stratifiée comporte pour chacune des différentes zones de température (12) un raccordement propre asservi (13) qui permet de charger et de décharger la zone de température (12) concernée.

5. Ensemble de stérilisation selon l'une des revendications précédentes, **caractérisé en ce que** la cuve (11) d'accumulation stratifiée est formée pour reprendre une colonne de fluide, les zones de température (12) étant disposées les unes au-dessus des autres ou les unes à côté des autres dans la colonne de fluide.

6. Ensemble de stérilisation selon la revendication 5, **caractérisé en ce qu'**au moins un ensemble de séparation (18) est disposé dans la cuve (11) d'accumulation stratifiée pour empêcher un mélange turbulent du fluide de différentes zones de température (12).

7. Ensemble de stérilisation selon l'une des revendications précédentes, **caractérisé en ce que** la cuve (11) d'accumulation stratifiée comporte au moins trois et de préférence au moins quatre zones de température (12) qui peuvent être chargées et déchargées séparément au moyen de raccordements (13) distincts.

8. Procédé de stérilisation recourant à un ensemble de stérilisation selon l'une des revendications précédentes et présentant les étapes suivantes :
- stérilisation de produits (4) dans une chambre (2) à l'aide d'eau chaude et/ou de vapeur chaude provenant d'un circuit primaire (3) de fluide,
- chauffage ou refroidissement sélectifs du circuit primaire (3) de fluide par un circuit secondaire (8) de fluide et
- conservation du fluide du deuxième circuit (8) dans différentes zones de température (12).

9. Procédé de stérilisation selon la revendication 8, **caractérisé en ce que** le fluide du circuit secondaire (8) provenant des différentes zones de température (12) est mélangé et/ou prélevé couche par couche pour chauffer et/ou refroidir le circuit primaire (3) de fluide.

10. Procédé de stérilisation selon l'une des revendications 8 ou 9, **caractérisé en ce que** le fluide accumulé de préférence dans la zone de température (12) présentant la température la plus basse peut être refroidi au moyen d'un circuit de refroidissement (15).
